# FASCICULE DE BREVET EUROPEEN

(11) **EP 0 857 480 B1**
(45) Date de publication et mention de la délivrance du brevet: **16.10.2002**
(21) Numéro de dépôt: 97400234.7
(22) Date de dépôt: 03.02.1997
(51) Int. Cl.: A61K 7/48

(54) **Utilisation du CO2 pour un traitement cosmétique des lipodystrophies segmentaires**
Verwendung von CO2 zur kosmetischen Behandlung von segmentären Lipodystrophien
Use of CO2 for the cosmetic treatment of segmental lipodystrophies

(43) Date de publication de la demande: 12.08.1998
(73) Titulaire: Régie Municipale des Eaux Minérales de Royat, 63130 Royat (FR)
(72) Inventeur: Bertrand, Annie, 63130 Royat (FR)
(74) Mandataire: Peuscet, Jacques

(56) Documents cités:
- FR-A- 2 732 216
- THERAPIE, vol. 50, no. 2, 1995, pages 113-122, XP002035212 R. FABRY: "Le traitement thermal des maladies artérielles: un placebo coûteux ou une thérapeutique à part entière?"

## Description

La présente invention concerne l'utilisation d'un gaz pour un traitement cosmétique des lipodystrophies segmentaires, encore communément appelées cellulite dans la suite du texte.

Il est bien connu que la cellulite ne présente généralement aucun risque pour la santé mais constitue un problème esthétique, en particulier pour les femmes. Les zones, dans lesquelles se développe la cellulite, se situent le plus souvent au niveau des cuisses, des genoux, de l'abdomen, des fesses et des hanches. La cellulite se traduit par un aspect irrégulier et granuleux de la peau, qui est inesthétique. De nombreux traitements cosmétiques sont proposés dans les instituts de beauté dans le but d'éliminer la cellulite, mais aucun ne donne de résultats vraiment satisfaisants.

Par ailleurs, on connaît un traitement thérapeutique de l'artérite nécessitant le contrôle d'un médecin, dans lequel on utilise un gaz thermal, en particulier le gaz thermal de Royat (France), contenant plus de 90 % en poids de CO₂. Dans ce but, on effectue des injections sous-cutanées à proximité des zones ischémiées des artères. L'injection se fait perpendiculairement à la peau dans la base de l'hypoderme. En pratique, on réalise une ou deux injections en amont et une à deux injections en aval de la zone ischémiée. Ensuite, on fait diffuser le gaz manuellement par massage en exerçant une pression vers le bas du corps. Par exemple, dans le cas de l'artérite de la jambe, on exerce une pression vers le pied. Généralement, on utilise pour les injections des aiguilles de 18 mm de longueur et de 0,6 mm de diamètre extérieur. On pique perpendiculairement au plan de la peau, à une profondeur variable selon la localisation de la zone ischémiée.

On a maintenant trouvé, selon la présente invention, qu'un gaz thermal, en particulier le gaz thermal de Royat (France), contenant plus de 90 % en poids de gaz carbonique (CO₂) pouvait également être utilisé de façon efficace pour un traitement cosmétique du corps humain en vue de l'élimination de la cellulite ; les modalités du traitement sont modifiées par rapport à celles du procédé de traitement thérapeutique de l'artérite.

La présente invention a pour premier objet l'utilisation d'un gaz thermal contenant plus de 90 % en poids de CO₂ pour le traitement cosmétique d'une zone de peau d'un sujet humain atteinte par la cellulite dans laquelle on injecte ledit gaz dans l'hypoderme au niveau de la masse cellulitique.

Le gaz thermal utilisé est, de préférence, le gaz thermal de Royat (France), ledit gaz ayant la composition pondérale moyenne suivante (en poids):
- CO₂ : 99,5 %
- O₂ : 1 0,10%
- N₂ : 0,35 %
- Argon
- Hélium ≤ 5.10⁻³ %
- Méthane
- Radon

Comme indiqué ci-dessus, contrairement à ce qui est effectué dans le cas du traitement de l'artérite, l'injection est effectuée dans l'hypoderme au niveau des masses cellulitiques, et non à la base de l'hypoderme.

Par ailleurs, dans la pratique, le traitement cosmétique selon l'invention se différencie avantageusement du traitement thérapeutique connu sur d'autres points.

Pour le traitement cosmétique de la cellulite, l'injection se fait de préférence avec une aiguille faisant un angle d'environ 30° par rapport à la surface libre de la peau. Lors de l'injection, l'évaluation de l'angle se fait généralement à l'oeil, et celui-ci peut varier sur une plage relativement grande entre 20 et 45°. L'essentiel est que l'injection ne soit pas faite perpendiculairement à la peau, ce qui a l'avantage de permettre, en retirant l'aiguille progressivement, d'augmenter la surface traitée par le gaz.

Pour le traitement cosmétique de la cellulite, on utilise avantageusement une aiguille de 25 mm de longueur et de 0,6 mm de diamètre extérieur.

Dans le traitement cosmétique de la cellulite, le volume de gaz injecté est, de préférence, par injection, d'environ 10 N cm³ sous une pression suffisante pour provoquer le gonflement de la zone injectée, généralement une pression relative de 0,2 x 10⁵ Pa. Dans ce cas, également, l'estimation du volume de gaz injecté peut être effectuée à l'oeil en observant le gonflement de la zone injectée.

Selon l'invention, on effectue de 1 à 5, de préférence 4 ou 5, injections par jour pendant environ 10 jours, sur une surface de peau d'environ 10 cm². Cette pratique sera dénommée le "traitement basique" dans la suite de la description. On peut effectuer simultanément le traitement basique de plusieurs zones de peau d'environ 10 cm², le nombre total d'injections effectuées par sujet traité pouvant aller jusqu'à 20.

Le traitement basique est cumulatif et le traitement basique peut être renouvelé plusieurs fois sur une même zone, en respectant un délai minimum de 7 jours entre deux traitements basiques.

De préférence, juste après les injections journalières, la peau est massée afin de répartir au mieux le gaz dans la zone traitée.

Le traitement de la cellulite selon l'invention peut être associé à un ou plusieurs autres traitements cosmétiques de la cellulite pour en augmenter l'efficacité.

La description ci-après, donnée à titre illustratif et non limitatif, permettra de mieux comprendre l'invention.

Pour commencer, on nettoie la zone de peau à traiter à l'aide d'un désinfectant, par exemple l'alcool.

Ensuite, on injecte ou insuffle le gaz provenant de la source thermale de Royat (France) à une température d'environ 28° à 30°C dans les zones atteintes de cellulite, grâce à un injecteur commercialisé sous la dénomination "LUER LOCK 6 mm" par la société "DCP MEDICAL" (France), auquel est fixée une aiguille de 25 mm de longueur et de 0,6 mm de diamètre extérieur.

Lors de l'injection, la peau est pincée entre le pouce et l'index et l'aiguille est introduite sur toute sa longueur avec un angle d'environ 30° par rapport à la surface de la peau. La quantité de gaz injectée est d'environ 10 N cm³ sous une pression relative de 0,2 x 10⁵ Pa pendant 15 à 20 secondes.

L'aiguille est retirée progressivement pendant la fin de l'injection afin de répartir le gaz sur une zone la plus importante possible.

Le traitement basique se fait sur une surface d'environ 10 cm² de peau à raison de 4 à 5 injections par jour pendant 10 jours.

Juste après les injections journalières, la peau est massée en surface avec un produit gras afin de répartir au mieux le gaz dans la zone traitée.

On a observé qu'un traitement de 10 jours sur 10 cm² de cuisse permet de réduire le volume de cellulite de façon perceptible à l'oeil, d'où une amélioration sensible de l'esthétique du sujet traité.

## Revendications

1. Utilisation d'un gaz thermal contenant plus de 90 % de CO₂ pour le traitement cosmétique d'une zone de peau d'un sujet humain atteinte par la cellulite dans laquelle on injecte ledit gaz dans l'hypoderme au niveau de la masse cellulitique.

2. Utilisation selon la revendication 1, **caractérisée par le fait que** le gaz thermal est le gaz thermal de Royat, France, ledit gaz ayant la composition pondérale moyenne suivante :
- CO₂ : 99,5 %
- O₂ : 1 0,10%
- N₂ : 0,35 %
- Argon
- Hélium ≤ 5.10⁻³ %
- Méthane
- Radon

3. Utilisation selon l'une des revendications 1 ou 2, **caractérisée par le fait que** l'injection se fait avec une aiguille faisant un angle d'environ 30° avec la surface libre de la peau.

4. Utilisation selon l'une des revendications 1 à 3, **caractérisée par le fait que** l'on utilise une aiguille de 25 mm de longueur et de 0,6 mm de diamètre extérieur.

5. Utilisation selon l'une des revendications 1 à 4, **caractérisée par le fait que** le volume de gaz injecté est, par injection, d'environ 10 N cm³ sous une pression relative d'environ 0,2 x 10⁵ Pascals.

6. Utilisation selon l'une des revendications 1 à 5, **caractérisée par le fait que** l'on effectue de 1 à 5 injections par jour pendant environ 10 jours pour une surface de peau d'environ 10 cm².

7. Utilisation selon l'une des revendications 1 à 6, **caractérisée par le fait que** l'on traite simultanément plusieurs zones de peau, le nombre total d'injections effectuées sur un sujet traité pouvant aller jusqu'à 20.

8. Utilisation selon l'une des revendications 1 à 7, **caractérisée par le fait qu'**elle est renouvelée plusieurs fois sur une même zone, en respectant un délai minimum de 7 jours entre deux traitements.

9. Utilisation selon l'une des revendications 1 à 8, **caractérisée par le fait que**, juste après les injections journalières, la peau est massée.

10. Utilisation selon l'une des revendications 1 à 10, **caractérisée par le fait qu'**elle est associée à un ou plusieurs autres traitements cosmétiques de la cellulite.

## Patentansprüche

1. Verwendung eines mehr als 90% CO₂ enthaltenden Thermalgases zur kosmetischen Behandlung einer Hautzone eines Menschen mit Cellulitis, wobei man auf dem Niveau der Cellulitismasse das Gas in die Hypodermis injiziert.

2. Verwendung nach Anspruch 1, **dadurch gekennzeichnet, daß** das Thermalgas das Thermalgas von Royat, Frankreich, ist, wobei das Gas folgende durchschnittliche Gewichtszusammen-setzung aufweist:
| | |
|---|---|
| **-CO**_{**2**} | **99,5%** |
| **-O**_{**2**} | **0.10%** |
| **-N**_{**2**} | **0.35%** |
| **-Argon** | |
| **-Helium** | **≤5.10**^{**-3**}**%** |
| **-Méthan** | |
| **-Radon** | |

3. Verwendung nach einem der Ansprüche 1 oder 2, **dadurch gekennzeichnet, daß** die Injektion mit einer Nadel vorgenommen wird, die mit der freien Oberfläche der Haut einen Winkel von etwa 30° bildet.

4. Verwendung nach einem der Ansprüche 1 bis 3, **dadurch gekennzeichnet, daß** man eine Nadel von 25 mm Länge und von 0,6 mm Außendurchmesser verwendet.

5. Verwendung nach einem der Ansprüche 1 bis 4, **dadurch. gekennzeichnet, daß** das Volumen des injizierten Gases, bei Injektion unter einem relativen Druck von etwa 0,2 x 10⁵ Pascal, etwa 10 N cm³ beträgt.

6. Verwendung nach einem der Ansprüche 1 bis 5, **dadurch gekennzeichnet, daß** man etwa 10 Tage lang 1 bis 5 Injektionen täglich für eine Hautoberfläche von etwa 10 cm² vornimmt.

7. Verwendung nach einem der Ansprüche 1 bis 6, **dadurch gekennzeichnet, daß** man mehrere Hautzonen gleichzeitig behandelt, wobei man insgesamt an einer zu behandelnden Person bis zu 20 Injektionen vornehmen kann.

8. Verwendung nach einem der Ansprüche 1 bis 7, **dadurch gekennzeichnet, daß** man sie an derselben Zone mehrfach erneuert, wobei man einen Mindestabstand von 7 Tagen zwischen beiden Behandlungen einhält.

9. Verwendung nach einem der Ansprüche 1 bis 8, **dadurch gekennzeichnet, daß** im Anschluß an die täglichen Injektionen die Haut massiert wird.

10. Verwendung nach einem der Ansprüche 1 bis 9, **dadurch gekennzeichnet, daß** man sie mit einer oder mehreren weiteren kosmetischen Behandlungen der Cellulitis verbindet.

## Claims

1. Use of a thermal gas containing more than 90% CO₂ for cosmetic treatment of an area of skin of a human subject suffering from cellulite, said gas being injected into the hypodermis in the area of the cellulite mass.

2. Use according to Claim 1, **characterized in that** the thermal gas is the thermal gas from Royat, France, said gas having the following average composition by weight:
- CO₂ : 99.5%
- O₂ : 0.10%
- N₂ : 1 0.35%
- N₂ : 0.35%
- argon
- helium ≤ 5.10⁻³ %
- methane
- radon

3. Use according to either of Claims 1 and 2, **characterized in that** the injection is carried out with a needle forming an angle of approximately 30° with the free surface of the skin.

4. Use according to one of Claims 1 to 3, **characterized in that** a needle is used which has a length of 25 mm and an external diameter of 0.6 mm.

5. Use according to one of Claims 1 to 4, **characterized in that** the volume of gas injected per injection is approximately 10 N cm³ at a relative pressure of approximately 0.2 x 10⁵ pascals.

6. Use according to one of Claims 1 to 5, **characterized in that** 1 to 5 injections are given per day, for about 10 days, for a skin surface area of approximately 10 cm².

7. Use according to one of Claims 1 to 6, **characterized in that** several areas of skin are treated simultaneously, and the total number of injections performed on a treated subject can be up to 20.

8. Use according to one of Claims 1 to 7, **characterized in that** it is repeated several times on the same area, observing a minimum period of 7 days between two treatments.

9. Use according to one of Claims 1 to 8, **characterized in that** the skin is massaged immediately after the daily injections.

10. Use according to one of Claims 1 to 9, **characterized in that** it is combined with one or more other cosmetic treatments of cellulite.
